# EUROPEAN PATENT SPECIFICATION

(11) **EP 4 491 104 B1**
(45) Date of publication and mention of the grant of the patent: **11.11.2026**
(21) Application number: 24182526.4
(22) Date of filing: 17.06.2024
(51) Int. Cl.: A61B 5/00, A43B 17/00, A43D 1/02, A61B 5/107

(54) **PREDICTION DEVICE, PREDICTION SYSTEM, AND PREDICTION METHOD**
VORHERSAGEVORRICHTUNG, VORHERSAGESYSTEM UND VORHERSAGEVERFAHREN
DISPOSITIF DE PRÉDICTION, SYSTÈME DE PRÉDICTION ET PROCÉDÉ DE PRÉDICTION

(30) Priority: 21.06.2023 JP 2023101825; 11.03.2024 JP 2024037478
(43) Date of publication of application: 15.01.2025
(73) Proprietor: ASICS Corporation, Kobe-shi, Hyogo 650-8555 (JP)
(72) Inventor: OKAMOTO, Toshiaki, Hyogo, 650-8555 (JP); HATANO, Genki, Hyogo, 650-8555 (JP)
(74) Representative: TBK

(56) References cited:
- US-A1- 2020 151 594
- US-A1- 2021 315 323

## Description

### BACKGROUND

### Technical field

The present disclosure relates to a prediction device, a prediction system, and a prediction method for predicting a foot shape of a subject.

### Background Information

Conventionally, a method of producing a shoe insole based on a foot shape in a low-load state is known (Japanese Patent JP 5717894 B). According to the method described in Japanese Patent JP 5717894 B, a shoe insole is produced based on a difference between data of a pressurized state obtained by measuring a foot shape of a subject standing on a transparent plate and data of a non-pressurized state obtained by measuring the foot shape of the subject that is lightly in contact with the transparent plate.

Furthermore, US 2020/151594 A1 discloses foot differentiation scoring pertaining to an individual's differences in their two feet. Thereby, the method includes receiving, from sensors of a scanning device, pressure and/or other measurement data corresponding to feet of a user, preprocessing the pressure and/or measurement data, and generating, based on the preprocessed pressure and/or measurement data, a foot differentiation score (FDS) based on differences between a left foot and a right foot of the user.

### SUMMARY

It is preferable to measure a foot shape when a custom-made shoe or shoe insole is to be produced or to choose a shoe or shoe insole that fits. However, since the measurement of the foot shape is generally performed by the subject in a standing posture, the foot shape in a loaded state in which a load is applied to a sole of a foot is measured. In order to produce a shoe or shoe insole more suitable for the foot shape, it is preferable to have information on the foot shape measured in an unloaded state in which almost no load is applied to the sole of the foot.

As the method for measuring the foot shape in the unloaded state, for example, there is known a method of molding and measuring the foot shape by winding a plaster bandage (cast) around the foot of the subject with the subject lying face down on a bed and pouring plaster into a hardened plaster bandage. However, according to this measurement method, a person having a skill to wrap a gypsum bandage around the foot so that no load is applied to the sole of the foot is required, and the foot shape in the unloaded state cannot be easily measured in a store or the like.

According to the method disclosed in Japanese Patent JP 5717894 B, it is necessary to measure the foot shape in a state where the subject stands on a transparent plate and further measure the foot shape in a state where the foot is lightly in contact with the transparent plate. In addition, according to this method, it is difficult for the subject to maintain a state of being lightly in contact with the transparent plate, and it is difficult to accurately measure the foot shape in a low-load state because the degree of contact with the transparent plate varies depending on the subject.

The present disclosure has been made to solve such a problem, and an object thereof is to provide a prediction device, a prediction system, and a prediction method capable of predicting the foot shape of the subject in different loaded states.

According to the invention, this object is achieved by a prediction device according to claim 1, a prediction system according to claim 11, and a prediction method according to claim 12. Further features and advantageous modifications are shown in the dependent claims.

A prediction device according to an aspect of the present disclosure predicts a foot shape of a subject. The prediction device includes: an input device that receives an input of measurement data of the foot shape of the subject in a first loaded state, a processing circuitry is using a prediction model trained by machine learning to predict the foot shape of the subject in a second loaded state in which a load is different from the first loaded state based on the measurement data received by the input device, and an output device that outputs prediction data of the foot shape of the subject in the second loaded state predicted by the processing circuitry. The prediction model is generated in advance through training by machine learning based on training measurement data of a foot shape of a training subject in the first loaded state and training shape data of the foot shape of the training subject in the second loaded state. The training measurement data and the training shape data are obtained from the training subject as three-dimensional data, and the prediction model is generated in advance through training by machine learning based on two-dimensional training data converted from the training measurement data and the training shape data respectively into two-dimensional data including information on the foot shape in a height direction of the training subject.

A prediction system according to an aspect of the present disclosure includes: a measurement device that measures the foot shape of the subject in the first loaded state; and the above-described prediction device.

A prediction method according to an aspect of the present disclosure predicts a foot shape of a subject. The prediction method includes: receiving an input of measurement data of the foot shape of the subject in a first loaded state, using a prediction model trained by machine learning, predicting the foot shape of the subject in a second loaded state in which the load is different from the first loaded state based on the measurement data, received, and outputting prediction data, which has been predicted, of the foot shape of the subject in the second loaded state. The prediction model is generated in advance through training by machine learning based on training measurement data of a foot shape of a training subject in the first loaded state and training shape data of the foot shape of the training subject in the second loaded state. The training measurement data and the training shape data are obtained from the training subject as three-dimensional data, and the prediction model is generated in advance through training by machine learning based on two-dimensional training data converted from the training measurement data and the training shape data respectively into two-dimensional data including information on the foot shape in a height direction of the training subject.

The foregoing and other objects, features, aspects and advantages of the present invention will become more apparent from the following detailed description of the present invention when taken in conjunction with the accompanying drawings.

### BRIEF DESCRIPTION OF THE DRAWINGS

Fig. 1 is a schematic diagram illustrating a configuration of a prediction system including a prediction device according to an embodiment.
Fig. 2 is a block diagram illustrating the configuration of the prediction device according to the embodiment.
Fig. 3 is a schematic diagram for illustrating a method of acquiring training data.
Fig. 4 is a flowchart for illustrating a process of a prediction method executed by the prediction device according to the embodiment.
Fig. 5 is a schematic diagram for illustrating a process of producing a shoe insole by the prediction system according to the embodiment.
Fig. 6 is a diagram schematically illustrating a processing of a prediction model used in the prediction device according to the embodiment.

### DETAILED DESCRIPTION

A prediction device according to an aspect of the present disclosure predicts a foot shape of a subject. The prediction device includes: an input device that receives an input of measurement data of the foot shape of the subject in a first loaded state, a processing circuitry is using a prediction model trained by machine learning to predict the foot shape of the subject in a second loaded state in which a load is different from the first loaded state based on the measurement data received by the input device, and an output device that outputs prediction data of the foot shape of the subject in the second loaded state predicted by the processing circuitry. Here, as an example, the prediction device, a prediction system, and a prediction method, which can predict the foot shape of the subject in a low-load state (second loaded state) in which the load is smaller than that of a loaded state (first loaded state) based on the foot shape of the subject in the loaded state, will be described. In particular, in the present embodiment, the prediction device, the prediction system, and the prediction method, which predict a foot shape in an unloaded state in which there is substantially no load based on the measurement data obtained by measuring the foot shape of the subject using the prediction model trained by machine learning to output prediction data, will be described. Training data is required to generate the prediction model trained by machine learning, and in order to generate a prediction model with high prediction accuracy, it is preferable that the training data is a set of training measurement data measured under the same measurement conditions and training shape data of the foot shape in the unloaded state acquired under the same acquisition conditions for a plurality of subjects.

Here, the measurement conditions for measuring the foot shape of the subject includes the posture of the subject such as a standing posture or a sitting posture, the state of the subject such as a resting state or an exercise state, and the like. The measurement conditions further include conditions such as the type of measurement device used for measurement and a measurement means using an image captured by a smartphone or the like.

The acquisition conditions for acquiring the foot shape in the unloaded state include whether the foot shape is molded and measured by winding a plaster bandage (cast) around the foot of the subject with the subject lying face down on a bed and pouring plaster into a hardened plaster bandage, whether the foot shape is measured in a sitting posture with the foot of the subject floating from the floor, and the like. Under these acquisition conditions, the foot shape in a substantial unloaded state can be acquired, and a slight load may be applied to the sole of the foot.

In the following description, training is performed by machine learning using training data, which is a set of training measurement data measured when the subject is in a standing posture and in a resting state and training shape data of a foot shape in the unloaded state acquired using a plaster bandage, to generate the prediction model. However, as long as the foot shape in the unloaded state can be predicted, data under different measurement conditions may be included in the training measurement data and data under different acquisition conditions may be included in the training shape data to be used as training data, and the prediction model may be generated through training by machine learning.

The foot shape predicted by the prediction device or the like is not limited to the foot shape in the unloaded state, and may be the foot shape of the subject in the low-load state in which the load is smaller than that of the loaded state. For example, the prediction model may be generated by performing training by machine learning using training data which is a set of training measurement data measured when the subject is in a sitting posture and in a resting state and training shape data of the foot shape of the subject who is lightly in contact with a transparent plate. Thus, it is possible to generate the prediction model for predicting the foot shape of the subject in the low-load state in which the load is smaller than that of the loaded state of the sitting posture based on the measurement data of the subject in the sitting posture. In the present disclosure, the low-load state includes the unloaded state in which there is substantially no load.

The prediction device, the prediction system, and the prediction method according to the embodiment will be described below with reference to the drawings. In the following description, the same configurations are denoted by the same reference signs. The names and functions of these configurations are also the same. Therefore, detailed description thereof will not be repeated.

### (Embodiment)

### [Configuration of Prediction System]

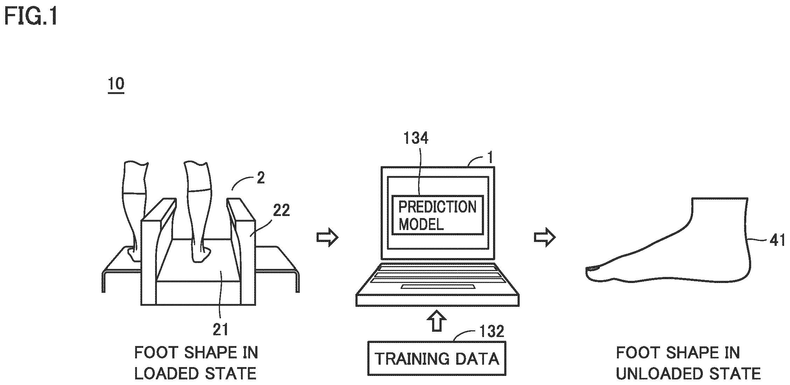

Fig. 1 is a schematic diagram illustrating a configuration of a prediction system 10 including a prediction device 1 according to the embodiment. The prediction system 10 includes a measurement device 2 that measures the foot shape of the subject in the loaded state (first loaded state), and the prediction device 1 that predicts the foot shape of the subject in the unloaded state (second loaded state) based on measurement data measured by the measurement device 2. The prediction device 1 uses a prediction model 134 trained by machine learning to predict the foot shape of the subject in the unloaded state. Therefore, the prediction device 1 is required to generate the prediction model 134 through training by machine learning using training data 132 before predicting the foot shape of the subject in the unloaded state. In the present embodiment, a configuration in which the prediction model 134 is generated by the prediction device 1 using the training data 132 will be described, but a configuration in which the prediction model 134 generated by another device is received by the prediction device 1 may also be used.

Hereinafter, the prediction device 1 will be described as a device that generates the prediction model 134 through training by machine learning using training data and predicts the foot shape of the subject in the unloaded state based on measurement data using the trained prediction model 134. The prediction device 1 outputs the predicted foot shape of the subject in the unloaded state as prediction data 41. It is possible to produce a shoe or shoe insole more suitable for the foot of the subject than a shoe or shoe insole produced in the foot shape in the loaded state by producing a custom-made shoe or shoe insole using the prediction data 41.

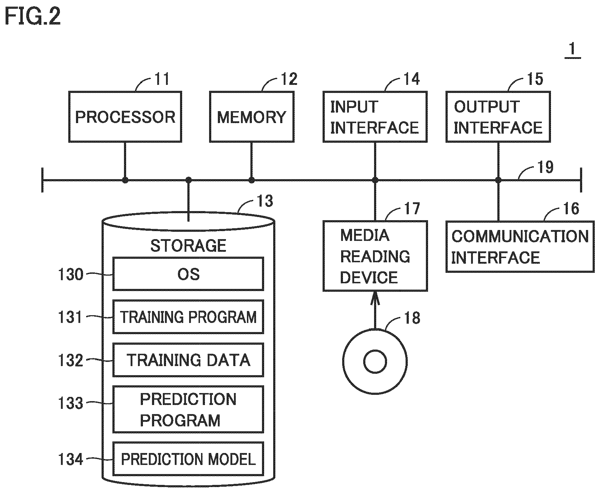

Fig. 2 is a block diagram illustrating the configuration of the prediction device 1 according to the embodiment. As illustrated in Fig. 2, the prediction device 1 includes a processor 11, a memory 12, a storage 13, an input interface 14, an output interface 15, a communication interface 16, and a media reading device 17. These configurations are connected to each other via a processor bus 19.

The processor 11 is a computer that reads programs (for example, an operating system (OS) 130, a training program 131, and a prediction program 133) stored in the storage 13, and expands the read programs in the memory 12 to execute the programs. The processor 11 is an example of "processing circuitry" and is configured of, for example, a central processing unit (CPU), a field programmable gate array (FPGA), a graphics processing unit (GPU), a multi processing unit (MPU), or the like. The processor 11 may be configured of a processing circuitry.

The memory 12 is configured of a volatile memory such as a dynamic random access memory (DRAM) or a static random access memory (SRAM), a nonvolatile memory such as a read only memory (ROM) or a flash memory, or the like.

The storage 13 is configured of, for example, a nonvolatile storage device such as a Hard Disk Drive (HDD) or a Solid State Drive (SSD). The storage 13 stores the training data 132, the prediction model 134, and the like in addition to the OS 130, the training program 131, and the prediction program 133.

The training program 131 generates the prediction model 134 through training by machine learning using the training data 132. The prediction program 133 predicts the foot shape in the unloaded state based on the measurement data of the foot shape of the subject in the loaded state using the prediction model 134. The prediction program 133 executes the prediction method by combining the training program 131 and the prediction model 134.

The input interface 14 is an example of input device, and receives an input operation of a keyboard, a mouse, a touch device, or the like. The output interface 15 is an example of output device, and outputs the prediction data 41 of the foot shape in the unloaded state predicted by the prediction device 1 to a display or the like.

The communication interface 16 is an example of input device and output device, and performs wired communication or wireless communication to receive input of measurement data from the measurement device 2 and to transmit and receive data to and from another device. The communication interface 16 can receive an input of measurement data and output the predicted prediction data 41 by transmitting and receiving data to and from another device. The communication interface 16 may receive an input of the training data 132 from another device, or may receive an image of the foot shape of the subject in the loaded state captured by a smartphone or the like. Note that, when the prediction device 1 receives the image, the prediction device 1 can obtain data (corresponding to measurement data) of the foot shape of the subject in the loaded state from the image based on a predetermined algorithm.

The media reading device 17 receives a storage medium such as a removable disk 18, a memory chip, or a USB memory, and acquires data stored in the removable disk 18, the memory chip, the USB memory, or the like. The media reading device 17 may read the training data 132 stored in the removable disk 18, or may store the predicted prediction data 41 in the removable disk 18 or the like and output the prediction data 41.

Note that, although it has been described that the prediction device 1 generates the prediction model 134 through training by machine learning using the training data 132, the prediction device 1 may receive the prediction model 134 from a server or the like via the communication interface 16 as long as the prediction model 134 generated by another device is prepared in advance. The prediction device 1 may read the prediction model 134 stored in the removable disk 18 or the like by the media reading device 17.

The measurement device 2 is, for example, a three-dimensional foot form scanner using laser measurement, and includes a top plate 21 and a laser measurement unit 22 installed in a manner of sandwiching the top plate 21. When the subject places his/her foot on the top plate 21 in a standing posture, a load is applied to the top plate 21 from the foot by the weight of the subject. That is, a load is applied to the foot of the subject. The measurement device 2 measures the foot shape while moving from the toe to the heel of the foot by the laser measurement unit 22 in a state where a load is applied to the foot of the subject. The measurement device 2 outputs measurement data (three-dimensional data) of the foot shape of the subject acquired by the laser measurement unit 22 to the prediction device 1. The measurement data may include at least the data of the foot shape acquired by the measurement device 2, and may include other data (for example, attribute information such as the gender or age of the subject).

### [Training Data]

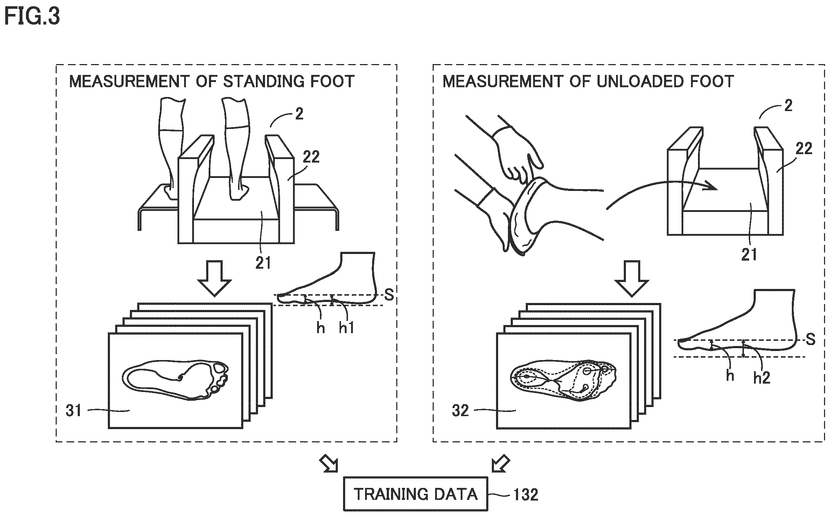

As the training data 132 for generating the prediction model 134 trained by machine learning, training data is used which is a set of training measurement data measured when the subject is in a standing posture and in a resting state (hereinafter, although not particularly described as in a resting state, the subject is assumed to be in a resting state at the time of measurement) and training shape data of the foot shape in the unloaded state acquired using a plaster bandage. Fig. 3 is a schematic diagram for illustrating a method of acquiring training data.

A plurality of subjects for training is prepared to obtain training data. The subjects for training may include a subject whose foot shape in the unloaded state is predicted. First, as illustrated in Fig. 3, the measurement of the standing foot is performed by the measurement device 2 for the plurality of subjects for training. That is, the foot of each training subject in a standing posture is measured using the measurement device 2 to acquire the foot shape of the training subject in the loaded state. When the training subject places his/her foot on the top plate 21 in a standing posture, the measurement device 2 measures the foot shape while moving from the toe to the heel of the foot by the laser measurement unit 22 in a state where a load is applied to the top plate 21 from the foot by the weight of the training subject. The data of the foot shape measured by the measurement device 2 is three-dimensional data. The three-dimensional data may be used as the training measurement data as it is, but in the present embodiment, the three-dimensional data is converted into two-dimensional data including information on the foot shape in the height direction, and the two-dimensional data after the conversion is used as training measurement data 31.

As illustrated in Fig. 3, the same plurality of subjects for training is measured for the unloaded foot using a plaster bandage, and data of the unloaded foot shape of the subjects for training is obtained. Specifically, the data of the foot shape of the subjects for training in the unloaded state is acquired by measuring the foot shape of the plaster, which is taken using the plaster bandage, by the measurement device 2. Therefore, the data of the foot shape obtained using the plaster bandage is three-dimensional data. The three-dimensional data may be used as the training shape data as it is, but in the present embodiment, the three-dimensional data is converted into two-dimensional data including information on the foot shape in the height direction, and the two-dimensional data after the conversion is used as training shape data 32.

Pair data of the training measurement data 31 and the training shape data 32 obtained from each of the plurality of subjects for training is adopted as the training data 132. The training data 132 converted into the two-dimensional data including information on the foot shape in the height direction is also referred to as two-dimensional training data. The training data 132 may be obtained by simply converting the training measurement data 31 and the training shape data 32, which are three-dimensional data, into the training measurement data 31 and the training shape data 32, which are two-dimensional data, and may also be only data in the vicinity of the sole of the foot, which is necessary for predicting the foot shape of the subject in the unloaded state. Therefore, the information on the foot shape in the height direction is, for example, information on a height h from a reference plane S to the sole of the foot as illustrated in Fig. 3. The reference plane S is, for example, a plane parallel to the floor surface in the standing posture, and is a plane at a predetermined height from the ankle or the instep of the training subject in the direction of the sole of the foot, a plane having the maximum outer shape, or the like. When the reference plane S is set inside the foot of the training subject as illustrated in Fig. 3, the height h from the reference plane S to the sole of the foot can be regarded as the depth from the reference plane S, and therefore the training measurement data 31 and the training shape data 32 converted into two-dimensional data are also referred to as depth image of the sole of the foot.

In addition, the training shape data 32 has larger irregularities of the sole of the foot than those of the training measurement data 31. Therefore, for the training measurement data 31, the data from the reference plane S to a height h1 (first height) of the training measurement data, which is three-dimensional data, is converted into two-dimensional data, while for the training shape data 32, the data from the reference plane S to a height h2 (second height (h1 < h2)) which is higher than the height h1 of the training shape data is converted into two-dimensional data.

When the training measurement data 31 and the training shape data 32 are converted into the depth image of the sole of the foot, respectively, the information on the height h from the reference plane S to the sole of the foot is converted into two-dimensional data as, for example, gray scale information (information of 256 gradations from white to black). Therefore, the depth image of the sole of the foot is an image in which the gray shading corresponding to the height h is applied to the two-dimensional foot shape. For example, the depth image of the sole of the foot expresses a portion where the height h is high in black, a portion where the height h is low in white, and a portion where the height h is intermediate in gray in the two-dimensional foot shape. There is no limitation on how to express the information on the height h in the depth image of the sole of the foot, and the information may be expressed not only as gradation information as described above but also as hue information or contour lines. For example, when the information on the height h is expressed as hue information, the depth image of the sole of the foot expresses a portion where the height h is high in red, a portion where the height h is low in blue, and a portion where the height h is intermediate in yellow in the two-dimensional foot shape. In any case, the training measurement data 31 and the training shape data 32 may be two-dimensional array data in which the information on the height h is stored as scalar value at each point of the two-dimensional foot shape.

Specifically, the training measurement data 31 represents an XY plane of 400 mm × 400 mm as a two-dimensional image of 256 pixels × 256 pixels, and represents the information on the height h stored in each pixel in a gray scale of 256 gradations, ranging the height of 0 mm to 20 mm inclusive from the reference plane S. A plane parallel to the floor surface in the standing posture is defined as the XY plane, and a direction perpendicular to the XY plane is defined as a Z direction, which is the direction of the height h. The training shape data 32 represents an XY plane of 400 mm × 400 mm as a two-dimensional image of 256 pixels × 256 pixels, and represents the information on the height h stored in each pixel in a gray scale of 256 gradations, ranging the height of 0 mm to 40 mm inclusive from the reference plane S. Note that the range of the training measurement data 31 stored as the information on the height h is 0 mm to 20 mm inclusive from the reference plane S and is narrower than that of the training shape data 32. However, the training measurement data 31 represents the information on the height h as the gray scale of 256 gradations, similarly to the training shape data 32. Therefore, the amount of information of the training measurement data 31 converted into two-dimensional data is the same as the amount of information of the training shape data 32 converted into two-dimensional data. Thus, the training measurement data 31 has a larger amount of data per 1 mm unit than the training shape data 32. Of course, the amount of data per 1 mm unit of the training measurement data 31 may be the same as the amount of data per 1 mm unit of the training shape data 32. The range from the reference plane S stored as the information on the height h in the training measurement data 31 and the training shape data 32 is not particularly limited, and may be changed depending on, for example, data (for example, shape and size) of the target foot.

In this way, the training data 132 can be used to generate a prediction model with high prediction accuracy while suppressing the amount of data to be processed by the prediction device 1 by converting the training measurement data 31 and the training shape data 32 into the depth image of the sole of the foot, respectively. Of course, the training data 132 may be three-dimensional data represented by a voxel, a point group, a mesh, a boundary surface, or the like, without converting the training measurement data 31 and the training shape data 32 respectively into the depth image of the sole of the foot. Since the prediction model is generated using the depth image of the sole of the foot as the training data 132, when the prediction device 1 predicts the foot shape in the unloaded state using the prediction model, the measurement data input to the prediction device 1 is required to be converted into the depth image of the sole of the foot. Since the foot shape in the unloaded state predicted by the prediction device 1 is output as the depth image of the sole of the foot, it is required to restore the foot shape in the unloaded state of the three-dimensional data from the output depth image of the sole of the foot.

### [Prediction Method]

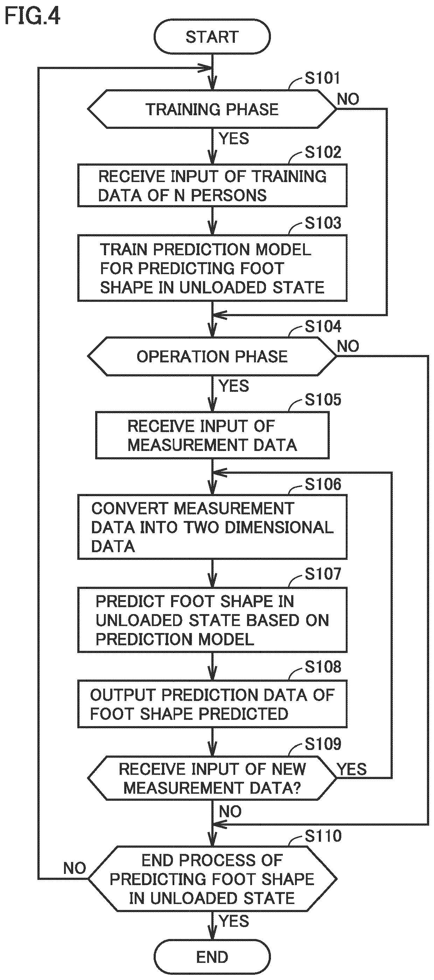

Next, the process of the prediction method executed by the prediction system 10 will be described with reference to a flowchart. Fig. 4 is a flowchart for illustrating the process of the prediction method executed by the prediction device 1 according to the embodiment. Each step illustrated in Fig. 4 is implemented by the processor 11 of the prediction device 1 executing the training program 131 and the prediction program 133.

First, the prediction device 1 determines whether or not to perform a process of a training phase by the training program 131 (step S101). When the prediction device 1 does not have the prediction model 134 yet or when the prediction model 134 has not been sufficiently trained, the prediction device 1 performs the process of the training phase. When the process of the training phase is not performed (NO in step S101), the prediction device 1 advances the process to step S104. On the other hand, when the process of the training phase is performed (YES in step S101), the prediction device 1 executes the training phase process of generating the prediction model 134 for predicting the foot shape of the subject in the unloaded state based on the measurement data of the foot shape of the subject in the loaded state.

In the training phase process, in order to generate the prediction model 134, the input of the training data 132 of N persons is received (step S102). The training data 132 received in step S102 includes the training measurement data 31 and the training shape data 32.

Next, the prediction device 1 generates the prediction model 134 for predicting the foot shape of the subject in the unloaded state through training by machine learning using the training data 132 (step S103). Here, the prediction model 134 includes, for example, a network structure such as a known neural network, support vector machine (SVM), or Bayesian network, and internal parameters used by the network structure, wherein the internal parameters are optimized (adjusted) based on the training shape data 32 and the prediction data of the foot shape in the unloaded state predicted from the training measurement data 31.

In particular, the training data 132 is pair data of the training measurement data 31 and the training shape data 32, and in the training by machine learning based on the pair data, an algorithm for performing image-to-image translation using the training shape data 32 as teacher data is used. Specifically, the algorithm used for conversion between pair data (pair images) as supervised learning may be convolutional auto-encoder (CAE), variational auto-encoder (VAE), U-Net, and the like, which are encoder-decoder models. Here, the variational auto-encoder (VAE) is a model in which a constraint is imposed so that latent variables of the convolution auto-encoder (CAE) have a normal distribution, and the U-Net is a model in which an encoder and a decoder of the convolution auto-encoder (CAE) are skip-connected. Note that convolution, vision transformer, or the like is used for the encoder, and transposed convolution or the like is used for the decoder.

Further, a generative adversarial network (GAN) model may be used as the algorithm used for conversion between pair data (pair images). As the GAN model, for example, pix2pix or the like is used. The algorithm used for the conversion between pair data (pair images) is not limited to the above-described algorithm, and an algorithm used for image generation, such as Flow-based model, diffusion model, autoregressive model (for example, pixelRNN, pixelCNN, ImageGPT, or the like), or energy-based model, can be appropriately used.

Next, the prediction device 1 determines whether or not to perform a process of an operation phase by the prediction program 133 (step S104). When the prediction model 134 sufficiently trained in the process of the training phase is generated, the prediction device 1 performs the process of the operation phase. When the process of the operation phase is not performed (NO in step S 104), the prediction device 1 advances the process to step S110. On the other hand, when the process of the operation phase is performed (YES in step S104), the prediction device 1 executes the operation phase process of predicting the foot shape of the subject in the unloaded state only based on the measurement data of the foot shape of the subject in the loaded state using the prediction model 134.

In the operation phase process, the input of the measurement data of the foot shape of the subject in the loaded state is received in order to predict the foot shape of the subject in the unloaded state (step S105). In step S105, only the measurement data of the foot shape of the subject measured by the measurement device 2 is required, and the foot shape in the unloaded state acquired using the plaster bandage is not required.

Next, the prediction device 1 converts the received measurement data, which is three-dimensional data, into two-dimensional data including information on the foot shape in the height direction so that the two-dimensional data can be used for the prediction model 134 (step S106). Specifically, the prediction device 1 converts the information on the height h from the reference plane S to the sole of the foot of the measurement data into the depth image of the sole of the foot as gray scale information.

Next, the prediction device 1 predicts the foot shape of the subject in the unloaded state using the prediction model 134 (step S107). The prediction device 1 converts the prediction data which is predicted in step S107 into three-dimensional data and outputs the three-dimensional data to a device for designing a shoe or shoe insole, or the like (step S108). Alternatively, the prediction device 1 may display the prediction data as an image on a display connected to the prediction device 1 for confirmation.

The prediction device 1 determines whether or not an input of new measurement data has been received from the measurement device 2 (step S109). When the input of the new measurement data has been received, the prediction device 1 predicts the foot shape of the subject in the unloaded state based on the new measurement data. Therefore, when the input of the new measurement data has been received (YES in step S109), the prediction device 1 returns the process to step S106.

On the other hand, when the input of the new measurement data has not been received (NO in step S109), the prediction device 1 determines whether or not to end the process of the prediction method (step S110). When the prediction device 1 has not received an input to end the process of the prediction method (NO in step S110), the prediction device 1 returns the process to step S101. On the other hand, when the prediction device 1 has received an input to end the process of the prediction method (YES in step S110), the prediction device 1 ends the process of the prediction method.

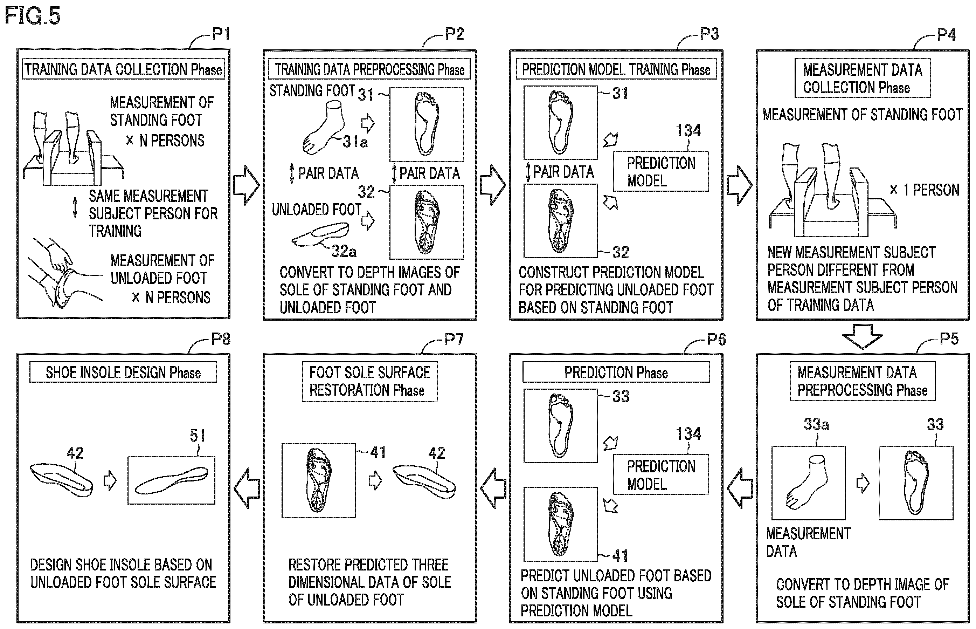

Next, an example of producing a shoe insole based on the foot shape of the subject in the unloaded state predicted by the prediction system 10 including at least the prediction device 1 and the measurement device 2 will be described with reference to schematic diagrams. Fig. 5 is a schematic diagram for illustrating the process of producing the shoe insole by the prediction system 10 according to the embodiment. First, in a training data collection phase P1 illustrated in Fig. 5, the measurement of the standing foot and the measurement of the unloaded foot using a plaster bandage are performed on N subjects for training by the measurement device 2 to collect the training data 132. Thus, N sets of pair data can be collected, each set of pair data being a combination of three-dimensional data of the foot shape and three-dimensional data of the foot shape in the unloaded state, which are obtained from the same training subject and measured by the measurement device 2.

In a training data preprocessing phase P2, three-dimensional data 31a of the foot shape and three-dimensional data 32a of the foot shape in the unloaded state, which are measured by the measurement device 2 and collected in the training data collection phase P1, are converted into two-dimensional data including information on the foot shape in the height direction, respectively. Specifically, the three-dimensional data 31a of the foot shape measured by the measurement device 2 is converted into the training measurement data 31 of the depth image of the sole of the foot, and the three-dimensional data 32a of the foot shape in the unloaded state is converted into the training shape data 32 of the depth image of the sole of the foot.

In a prediction model training phase P3, the prediction model 134 trained by machine learning is generated using N sets of pair data, each set being a combination of the training measurement data 31 and the training shape data 32. The process from the training data collection phase P1 to the prediction model training phase P3 corresponds to the process of the training phase illustrated in Fig. 4.

Next, in a measurement data collection phase P4, measurement data 33a of the subject (who may be different from or the same as the training subject) whose foot shape in the unloaded state is to be predicted is collected. The foot shape of the subject in the standing posture and in the loaded state is acquired as the measurement data 33a using the measurement device 2. The measurement data 33a is three-dimensional data.

In a measurement data preprocessing phase P5, the measurement data 33a, which is the three-dimensional data collected in the measurement data collection phase P4, is converted into two-dimensional data including information on the foot shape in the height direction. Specifically, the three-dimensional data of the measured foot shape is converted into the measurement data 33 which is the depth image of the sole of the foot.

In a prediction phase P6, the prediction model 134 is used to predict the foot shape of the subject in the unloaded state based on the measurement data 33 of the depth image of the sole of the foot. The prediction data 41 which is predicted in the prediction phase P6 is shape data of the depth image of the sole of the foot, and is two-dimensional data including information on the foot shape in the height direction. The process from the measurement data collection phase P4 to the prediction phase P6 corresponds to the process of the operation phase illustrated in Fig. 4.

In a foot sole surface restoration phase P7, foot sole shape data 42, which is three-dimensional data, is restored from the prediction data 41, which is two-dimensional data including information on the foot shape in the height direction, so as to be easily used for designing the shoe insole. The foot sole shape data 42 is shape data, which is three-dimensional data from the reference plane S to the sole of the foot. In the foot sole surface restoration phase P7, the shape data of the ankle side from the reference plane S may be extracted from the measurement data 33a, which is three-dimensional data, and combined with the foot sole shape data 42 to restore the foot sole shape data of the entire foot.

In a shoe insole design phase P8, design data 51 of the shoe insole is designed based on the foot sole shape data 42 restored in the foot sole surface restoration phase P7. In Fig. 5, the design of the shoe insole has been described as an example, but the design data of the shoe may be designed based on the foot sole shape data 42 or the foot sole shape data of the entire foot.

### [Prediction Model]

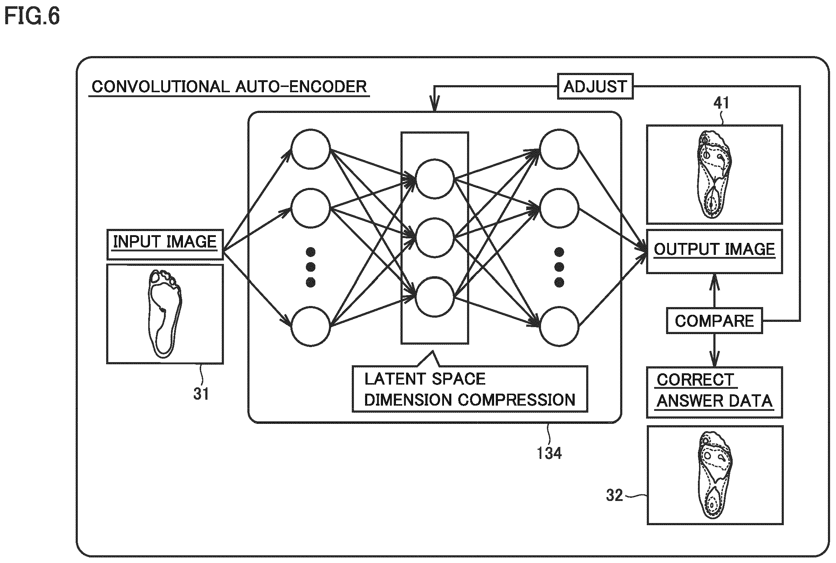

Next, the prediction model 134 will be described with reference to schematic diagrams. Fig. 6 is a diagram schematically illustrating a processing of the prediction model used in the prediction device 1 according to the embodiment. For the prediction model 134, a convolutional auto-encoder (CAE) is used, and the depth image of the sole of the foot of the training measurement data 31 is used as an input image, and the prediction data 41 of the foot shape of the subject in the unloaded state is used as an output image. Here, the convolutional auto-encoder is a multi-layer neural network including a structure for performing encoding by one or more convolutional layers or pooling layers and a structure for performing decoding by one or more de-convolutional layers.

Although it has been described that the convolutional auto-encoder (CAE), which is an encoder-decoder model, is used as the prediction model 134, a vision transformer, for example, may be used as an encoder instead of convolution. When a vision transformer is used as an encoder, the model has a structure without a pooling layer as in convolution, and thus, the contrast between the predicted foot shape of the subject and the background becomes relatively clear in the output image of the prediction data 41 of the foot shape of the subject in the unloaded state, the predicted outline shape of the foot is less likely to be disturbed, and noise is less likely to be generated in a part of the background.

Further, for the prediction model 134, a generative adversarial network (GAN) model may be used instead of the encoder-decoder model, and for example, pix2pix may be used. When pix2pix is used for the prediction model 134, the contrast between the predicted foot shape of the subject and the background becomes relatively clear compared to the convolution auto-encoder (CAE) using convolution for the encoder, because the model similarly has a structure without a pooling layer as in convolution.

Training is performed in which the depth image of the sole of the foot of the training shape data 32 is used as correct answer data, the output image of the predicted prediction data 41 is compared with the correct answer data, and the prediction model 134 is adjusted so that a loss function between the output image and the correct answer data is minimized. Therefore, the prediction device 1 can predict the foot shape of the subject in the unloaded state based on the foot shape of the subject in the loaded state measured by the measurement device 2 using the trained prediction model 134. Thus, the prediction device 1 can obtain the foot shape of the subject in the unloaded state without using a plaster bandage by using the trained prediction model 134.

### [Modifications]

(1) In the above-described embodiment, the training data 132 is collected from a plurality of subjects selected regardless of the attribute (for example, gender, age, race, or the like), and the prediction model 134 is generated through training by machine learning using the collected training data 132. The present invention is not limited thereto, and training data may be collected from a plurality of subjects separately for each attribute, and a prediction model may be generated through training by machine learning using the training data separated for each attribute. The prediction device 1 has a prediction model for each attribute, and receives attribute information of the subject to select a prediction model corresponding to the attribute. Then, the prediction device 1 predicts the foot shape of the subject in the unloaded state based on the measurement data using the selected prediction model. Thus, the prediction device 1 can accurately predict the foot shape of the subject in the unloaded state by selecting a prediction model with high prediction accuracy based on the attribute information of the subject.
(2) In the above-described embodiment, the prediction of the foot shape of the subject in the unloaded state based on the measurement data of the foot shape of the subject in the loaded state using the trained prediction model has been described. However, the state in which the foot shape is predicted is not limited to the unloaded state. For example, the foot shape of the subject in a low-load state in which the load is not zero may be predicted using a prediction model through training by machine learning using the training shape data of the foot shape of the training subject in the low-load state in which the load is smaller than that in the loaded state. Further, the foot shape of the subject in a high-load state may be predicted using a prediction model through training by machine learning using the training shape data of the foot shape of the training subject in the high-load state in which the load is larger than that in the loaded state. That is, the prediction device 1 can predict the foot shape of the subject in different loaded states by generating a prediction model through training by machine learning based on the training measurement data of the foot shape of the training subject in the first loaded state and the training shape data of the foot shape of the training subject in the second loaded state in which the load is different from the first loaded state.

## Claims

1. A prediction device (1) for predicting a foot shape of a subject, the prediction device (1) comprising:
an input device (14) that receives an input of measurement data of the foot shape of the subject in a first loaded state; and
a processing circuitry (11) using a prediction model (134) trained by machine learning to predict the foot shape of the subject in a second loaded state in which a load is different from the first loaded state based on the measurement data received by the input device (14),
**characterized by**
an output device (15) that outputs prediction data (41) of the foot shape of the subject in the second loaded state predicted by the processing circuitry (11), wherein
the prediction model (134) is generated in advance through training by machine learning based on training measurement data (31) of a foot shape of a training subject in the first loaded state and training shape data (32) of the foot shape of the training subject in the second loaded state,
the training measurement data (31) and the training shape data (32) are obtained from the training subject as three-dimensional data, and
the prediction model (134) is generated in advance through training by machine learning based on two-dimensional training data converted from the training measurement data (31) and the training shape data (32) respectively into two-dimensional data including information on the foot shape in a height direction of the training subject.

2. The prediction device (1) according to claim 1, wherein the two-dimensional training data includes the information on the foot shape in the height direction of the training subject as gradation information.

3. The prediction device (1) according to claim 1 or 2, wherein the information on the foot shape in the height direction is information on a height from a reference plane to a sole of a foot.

4. The prediction device (1) according to claim 3, wherein in the two-dimensional training data, data from the reference plane to a first height in a direction of the sole of the foot is converted from the training measurement data (31) into two-dimensional data, and data from the reference plane to a second height higher than the first height in the direction of the sole of the foot is converted from the training shape data (32) into two-dimensional data.

5. The prediction device (1) according to claim 4, wherein an amount of information of the training measurement data (31) converted into two-dimensional data is same as an amount of information of the training shape data (32) converted into two-dimensional data.

6. The prediction device (1) according to any one of claims 1 to 5, wherein the processing circuitry (11) predicts the foot shape of the subject in the second loaded state based on the measurement data, which has been converted into two-dimensional data, using the prediction model (134).

7. The prediction device (1) according to claim 6, wherein the processing circuitry (11) restores the prediction data (41) which is two-dimensional data to three-dimensional data.

8. The prediction device (1) according to any one of claims 1 to 7, wherein
the prediction model (134) is generated in advance through training by machine learning for each attribute information of the training subject, and
the input device (14) receives the at least one of attribute information of the subject, and use the prediction model (134) corresponding to the inputted attribute to predict the foot shape of the subject in the second loaded state based on the measurement data received by the input device (14).

9. The prediction device (1) according to any one of claims 1 to 8, wherein
the foot shape is a foot sole shape.

10. The prediction device (1) according to any one of claims 1 to 8, wherein
the training measurement data (31) and the training shape data (32) is a paired image data.

11. A prediction system comprising:
a measurement device (2) that measures the foot shape of the subject in the first loaded state; and
the prediction device (1) according to any one of claims 1 to 10.

12. A computer-implemented prediction method for predicting a foot shape of a subject, the prediction method comprising:
receiving an input of measurement data of the foot shape of the subject in a first loaded state;
using a prediction model (134) trained by machine learning; and
predicting the foot shape of the subject in a second loaded state in which a load is different from the first loaded state based on the measurement data received,
**characterized by**
outputting prediction data (41), which has been predicted, of the foot shape of the subject in the second loaded state, wherein
the prediction model (134) is generated in advance through training by machine learning based on training measurement data (31) of a foot shape of a training subject in the first loaded state and training shape data (32) of the foot shape of the training subject in the second loaded state,
the training measurement data (31) and the training shape data (32) are obtained from the training subject as three-dimensional data, and
the prediction model (134) is generated in advance through training by machine learning based on two-dimensional training data converted from the training measurement data (31) and the training shape data (32) respectively into two-dimensional data including information on the foot shape in a height direction of the training subject.

## Patentansprüche

1. Vorhersagevorrichtung (1) zum Vorhersagen einer Fußform eines Subjekts, wobei die Vorhersagevorrichtung (1) aufweist:
eine Eingabeeinrichtung (14), die eine Eingabe von Messdaten der Fußform des Subjekts in einem ersten Belastungszustand empfängt; und
eine Verarbeitungsschaltung (11), die ein durch maschinelles Lernen trainiertes Vorhersagemodell (134) verwendet, um die Fußform des Subjekts in einem zweiten Belastungszustand vorherzusagen, in dem sich eine Belastung von derjenigen im ersten Belastungszustand unterscheidet, basierend auf den von der Eingabeeinrichtung (14) empfangenen Messdaten,
**gekennzeichnet durch**
eine Ausgabeeinrichtung (15), die durch die Verarbeitungsschaltung (11) vorhergesagte Vorhersagedaten (41) der Fußform des Subjekts im zweiten Belastungszustand ausgibt, wobei
das Vorhersagemodell (134) im Voraus durch Training mittels maschinellen Lernens auf der Grundlage von Trainingsmessdaten (31) einer Fußform eines Trainingssubjekts im ersten Belastungszustand und Trainingsformdaten (32) der Fußform des Trainingssubjekts im zweiten Belastungszustand erzeugt wird,
die Trainingsmessdaten (31) und die Trainingsformdaten (32) vom Trainingssubjekt als dreidimensionale Daten erhalten werden, und
das Vorhersagemodell (134) im Voraus durch Training mittels maschinellen Lernens auf der Grundlage zweidimensionaler Trainingsdaten erzeugt wird, die jeweils aus den Trainingsmessdaten (31) und den Trainingsformdaten (32) in zweidimensionale Daten umgewandelt werden und Informationen über die Fußform des Trainingssubjekts in einer Höhenrichtung enthalten.

2. Vorhersagevorrichtung (1) gemäß Anspruch 1, wobei die zweidimensionalen Trainingsdaten die Informationen über die Fußform des Trainingssubjekts in der Höhenrichtung als Abstufungsinformationen enthalten.

3. Vorhersagevorrichtung (1) gemäß Anspruch 1 oder 2, wobei die Informationen über die Fußform in der Höhenrichtung Informationen über eine Höhe von einer Bezugsebene bis zu einer Fußsohle sind.

4. Vorhersagevorrichtung (1) gemäß Anspruch 3, wobei in den zweidimensionalen Trainingsdaten Daten von der Bezugsebene bis zu einer ersten Höhe in Richtung der Fußsohle aus den Trainingsmessdaten (31) in zweidimensionale Daten umgewandelt werden und Daten von der Bezugsebene bis zu einer zweiten Höhe, die höher als die erste Höhe ist, in Richtung der Fußsohle aus den Trainingsformdaten (32) in zweidimensionale Daten umgewandelt werden.

5. Vorhersagevorrichtung (1) gemäß Anspruch 4, wobei eine Informationsmenge der in zweidimensionale Daten umgewandelten Trainingsmessdaten (31) gleich einer Informationsmenge der in zweidimensionale Daten umgewandelten Trainingsformdaten (32) ist.

6. Vorhersagevorrichtung (1) gemäß einem der Ansprüche 1 bis 5, wobei die Verarbeitungsschaltung (11) die Fußform des Subjekts im zweiten Belastungszustand auf der Grundlage der in zweidimensionale Daten umgewandelten Messdaten unter Verwendung des Vorhersagemodells (134) vorhersagt.

7. Vorhersagevorrichtung (1) gemäß Anspruch 6, wobei die Verarbeitungsschaltung (11) die Vorhersagedaten (41), bei denen es sich um zweidimensionale Daten handelt, in dreidimensionale Daten rückumwandelt.

8. Vorhersagevorrichtung (1) gemäß einem der Ansprüche 1 bis 7, wobei
das Vorhersagemodell (134) im Voraus durch Training mittels maschinellen Lernens für jede Attributinformation des Trainingssubjekts erzeugt wird, und
die Eingabeeinrichtung (14) mindestens eine Attributinformation des Subjekts empfängt und das Vorhersagemodell (134), das dem eingegebenen Attribut entspricht, verwendet, um die Fußform des Subjekts im zweiten Belastungszustand auf der Grundlage der von der Eingabeeinrichtung (14) empfangenen Messdaten vorherzusagen.

9. Vorhersagevorrichtung (1) gemäß einem der Ansprüche 1 bis 8, wobei
die Fußform eine Fußsohlenform ist.

10. Vorhersagevorrichtung (1) gemäß einem der Ansprüche 1 bis 8, wobei
die Trainingsmessdaten (31) und die Trainingsformdaten (32) gepaarte Bilddaten sind.

11. Vorhersagesystem, mit:
einer Messvorrichtung (2), die die Fußform des Subjekts im ersten Belastungszustand misst; und
der Vorhersagevorrichtung (1) gemäß einem der Ansprüche 1 bis 10.

12. Computerimplementiertes Vorhersageverfahren zum Vorhersagen einer Fußform eines Subjekts, wobei das Vorhersageverfahren aufweist:
Empfangen einer Eingabe von Messdaten der Fußform des Subjekts in einem ersten Belastungszustand;
Verwenden eines durch maschinelles Lernen trainierten Vorhersagemodells (134); und
Vorhersagen der Fußform des Subjekts in einem zweiten Belastungszustand, in dem sich eine Belastung von derjenigen im ersten Belastungszustand unterscheidet, auf der Grundlage der empfangenen Messdaten,
**gekennzeichnet durch**
Ausgeben vorhergesagter Vorhersagedaten (41) der Fußform des Subjekts im zweiten Belastungszustand, wobei
das Vorhersagemodell (134) im Voraus durch Training mittels maschinellen Lernens auf der Grundlage von Trainingsmessdaten (31) einer Fußform eines Trainingssubjekts im ersten Belastungszustand und Trainingsformdaten (32) der Fußform des Trainingssubjekts im zweiten Belastungszustand erzeugt wird,
die Trainingsmessdaten (31) und die Trainingsformdaten (32) vom Trainingssubjekt als dreidimensionale Daten erhalten werden, und
das Vorhersagemodell (134) im Voraus durch Training mittels maschinellen Lernens auf der Grundlage zweidimensionaler Trainingsdaten erzeugt wird, die jeweils aus den Trainingsmessdaten (31) und den Trainingsformdaten (32) in zweidimensionale Daten umgewandelt werden und Informationen über die Fußform des Trainingssubjekts in einer Höhenrichtung enthalten.

## Revendications

1. Dispositif de prédiction (1) pour prédire une forme de pied d'un sujet, le dispositif de prédiction (1) comprenant :
un dispositif d'entrée (14) qui reçoit une entrée de données de mesure de la forme de pied du sujet dans un premier état chargé ; et
un circuit de traitement (11) utilisant un modèle de prédiction (134) entraîné par apprentissage automatique à prédire la forme de pied du sujet dans un second état chargé dans lequel une charge est différente du premier état chargé sur la base des données de mesure reçues par le dispositif d'entrée (14),
**caractérisé par**
un dispositif de sortie (15) qui fournit en sortie des données de prédiction (41) de la forme de pied du sujet dans le second état chargé prédit par le circuit de traitement (11), dans lequel
le modèle de prédiction (134) est généré à l'avance par le biais d'entraînement par apprentissage automatique sur la base de données de mesure d'entraînement (31) d'une forme de pied d'un sujet d'entraînement dans le premier état chargé et de données de forme d'entraînement (32) de la forme de pied du sujet d'entraînement dans le second état chargé,
les données de mesure d'entraînement (31) et les données de forme d'entraînement (32) sont obtenues à partir du sujet d'entraînement en tant que données tridimensionnelles, et
le modèle de prédiction (134) est généré à l'avance par le biais d'entraînement par apprentissage automatique sur la base de données d'entraînement bidimensionnelles converties à partir des données de mesure d'entraînement (31) et des données de forme d'entraînement (32) respectivement en données bidimensionnelles incluant des informations sur la forme de pied dans le direction de la hauteur du sujet d'entraînement.

2. Dispositif de prédiction (1) selon la revendication 1, dans lequel les données d'entraînement bidimensionnelles incluent les informations sur la forme de pied dans la direction de la hauteur du sujet d'entraînement en tant qu'informations de gradation.

3. Dispositif de prédiction (1) selon la revendication 1 ou la revendication 2, dans lequel les informations sur la forme de pied dans la direction de la hauteur sont des informations sur une hauteur d'un plan de référence à une semelle d'un pied.

4. Dispositif de prédiction (1) selon la revendication 3, dans lequel dans les données d'entraînement bidimensionnelles, des données du plan de référence à une première hauteur dans une direction de la semelle du pied sont converties à partir des données de mesure d'entraînement (31) en données bidimensionnelles, et des données du plan de référence à une seconde hauteur supérieure à la première hauteur dans la direction de la semelle du pied sont converties à partir des données de forme d'entraînement (32) en données bidimensionnelles.

5. Dispositif de prédiction (1) selon la revendication 4, dans lequel une quantité d'informations des données de mesure d'entraînement (31) converties en données bidimensionnelles est identique à une quantité d'informations des données de forme d'entraînement (32) converties en données bidimensionnelles.

6. Dispositif de prédiction (1) selon l'une quelconque des revendications 1 à 5, dans lequel le circuit de traitement (11) prédit la forme de pied du sujet dans le second état chargé sur la base des données de mesure, qui ont été converties en données bidimensionnelles, en utilisant le modèle de prédiction (134).

7. Dispositif de prédiction (1) selon la revendication 6, dans lequel le circuit de traitement (11) restaure les données de prédiction (41) qui sont des données bidimensionnelles en données tridimensionnelles.

8. Dispositif de prédiction (1) selon l'une quelconque des revendications 1 à 7, dans lequel
le modèle de prédiction (134) est généré à l'avance par le biais d'entraînement par apprentissage automatique pour chaque information d'attribut du sujet d'entraînement, et
le dispositif d'entrée (14) reçoit l'au moins une information d'attribut du sujet, et utilise le modèle de prédiction (134) correspondant à l'attribut entré pour prédire la forme de pied du sujet dans le second état chargé sur la base des données de mesure reçues par le dispositif d'entrée (14).

9. Dispositif de prédiction (1) selon l'une quelconque des revendications 1 à 8, dans lequel la forme de pied est une forme de semelle de pied.

10. Dispositif de prédiction (1) selon l'une quelconque des revendications 1 à 8, dans lequel les données de mesure d'entraînement (31) et les données de forme d'entraînement (32) sont des données d'image appariées.

11. Système de prédiction comprenant :
un dispositif de mesure (2) qui mesure la forme de pied du sujet dans le premier état chargé ; et
le dispositif de prédiction (1) selon l'une quelconque des revendications 1 à 10.

12. Procédé de prédiction
mise en œuvre par ordinateur pour prédire une forme de pied d'un sujet, le procédé de prédiction comprenant :
la réception d'une entrée de données de mesure de la forme de pied du sujet dans un premier état chargé ;
l'utilisation d'un modèle de prédiction (134) entraîné par apprentissage automatique ; et
la prédiction de la forme de pied du sujet dans un second état chargé dans lequel une charge est différente du premier état chargé sur la base des données de mesure reçues,
**caractérisé par**
la fourniture en sortie de données de prédiction (41), qui ont été prédites, de la forme de pied du sujet dans le second état chargé, dans lequel
le modèle de prédiction (134) est généré à l'avance par le biais d'entraînement par apprentissage automatique sur la base de données de mesure d'entraînement (31) d'une forme de pied d'un sujet d'entraînement dans le premier état chargé et de données de forme d'entraînement (32) de la forme de pied du sujet d'entraînement dans le second état chargé,
les données de mesure d'entraînement (31) et les données de forme d'entraînement (32) sont obtenues à partir du sujet d'entraînement en tant que données tridimensionnelles, et
le modèle de prédiction (134) est généré à l'avance par le biais d'entraînement par apprentissage automatique sur la base de données d'entraînement bidimensionnelles converties à partir des données de mesure d'entraînement (31) et des données de forme d'entraînement (32) respectivement en données bidimensionnelles incluant des informations sur la forme de pied dans le direction de la hauteur du sujet d'entraînement.
